# EUROPEAN PATENT SPECIFICATION

(11) **EP 2 237 683 B1**
(45) Date of publication and mention of the grant of the patent: **11.11.2015**
(21) Application number: 09704236.0
(22) Date of filing: 22.01.2009
(51) Int. Cl.: A23L 1/226, C07C 69/017

(54) **METHOD OF FLAVORING**
AROMATISIERUNGSVERFAHREN
PROCÉDÉ D'AROMATISATION

(30) Priority: 22.01.2008 US 11818
(43) Date of publication of application: 13.10.2010
(73) Proprietor: Givaudan SA, 1214 Vernier (CH)
(72) Inventor: YANG, Xiaogen, West Chester Ohio 45069 (US); WANG, Yili, Mason Ohio 45040 (US); EILERMAN, Robert, G., Manhasset New York 11030 (US); JORDAN, Jason, Cincinnati Ohio 45244 (US)
(74) Representative: McStea, John Anthony
(86) International application number: PCT/CH2009/000026
(87) International publication number: WO 2009/092176

(56) References cited:
- EP-A1- 0 870 754
- EP-A2- 0 470 765
- EP-A2- 0 470 766
- WO-A1-2008/105652
- US-A- 655 208
- US-A- 2 078 205
- US-A- 4 533 746
- US-A- 5 358 930
- US-A1- 2006 045 954
- DIKUSAR E A ET AL: "Preparative Synthesis of Vanillin and Vanillal Alkanoates" RUSSIAN JOURNAL OF APPLIED CHEMISTRY, NAUKA/INTERPERIODICA, MO, vol. 78, no. 1, 1 January 2005 (2005-01-01), pages 120-124, XP019299400 ISSN: 1608-3296
- NIEDERAUER T: "Eigenschaften und Anwendung von Geschmacksverstärkern" DMZ. LEBENSMITTELINDUSTRIE UND MILCHWISSENSCHAFT, VOLKSWIRTSCHAFTLICHER VERLAG GMBH. MUNCHEN, DE, vol. 115, no. 7, 1 January 1994 (1994-01-01), pages 324-330, XP009039658 ISSN: 0938-9369
- DATABASE BEILSTEIN [Online] BEILSTEIN INSTITUTE FOR ORGANIC CHEMISTRY, FRANKFURT-MAIN, DE; Substance Identification "CAS Registry Number 66475-96-5" XP002528222 Database accession no. 2532721 (BRN)

## Description

This disclosure relates to the achievement of vanilla beany flavor.

The achievement of a desirable vanilla beany flavor has long been a desire of flavorists. Hitherto it has not been possible to achieve by means other than using the natural material.

It has now been discovered that it is possible to provide such a desirable vanilla beany flavor. There is therefore provided a method of providing vanilla beany flavor in an orally-receivable product, comprising adding to a product base at least one compound selected from the group consisting of those according to Formula I 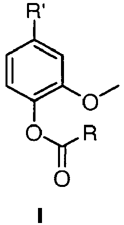 in which R' is selected from the group consisting of hydrogen and methyl, and R is selected from the group consisting of hydrogen, methyl, ethyl, propyl, isopropyl, n-butyl, 1- and 2-methylpropyl, n-pentyl, 1-, 2- and 3-methylbutyl, 1-, 2-, 3-, and 4-methylpentyl, 1-methylbut-2-ene, hydroxyethyl, ethylcarboxy, 2-tetrahydrofuranyl and a group of Formula VII.

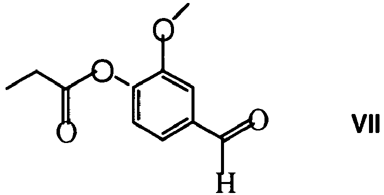

By "product base" is meant any combination of two or more materials that are combined with suitable flavor ingredients to produce a product that is orally-receivable, that is taken orally, either for ingesting (such as a foodstuff or a beverage) or for spitting (such as a mouthwash). All the standard materials known to the art may be used, and the skilled person will in every case be able to select suitable materials.

Some of the compounds of Formula I above are novel materials. There is therefore also provided a compound selected from compounds according to Formulae III-IV, in which R has the value shown:

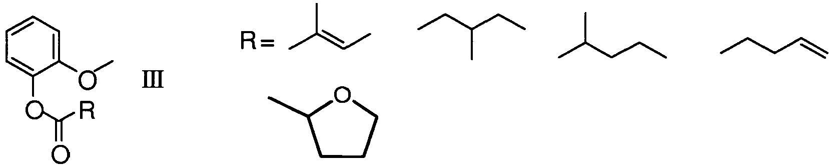

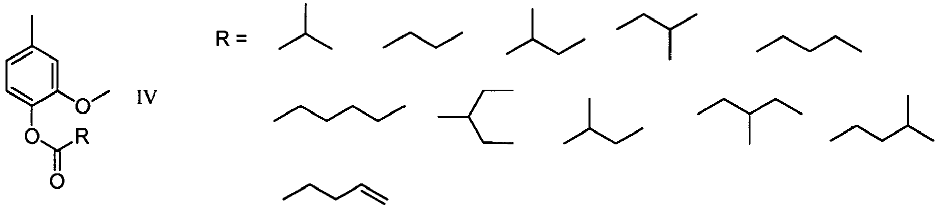

The compounds of Formula I may be prepared by standard methods known to the art, and the skilled person will be able to do so. For example, guaiacol and p-creosol esters may be prepared according to the methods described in the publication by Parish R. C. and Stock L. M. in, J. Org. Chem., (1965) 30(3), 927-9, and vanillin and maltol esters may be prepared by the following methods: 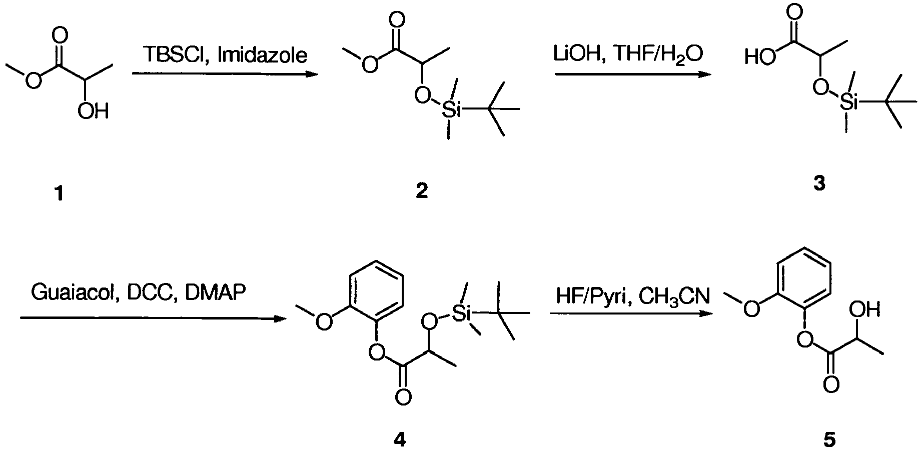 Particular examples of compounds that confer beany flavor are shown in the following tables. Table 1 contains particularly efficacious compounds, Table 2 less efficacious compounds and Table 3 still less efficacious compounds.

**Table 1**

| **Common Name** | **Chemical Name** | **CAS** | **Structure** |
|---|---|---|---|
| Guaiacol isovalerate | 2-methoxyphenyl 3-methylbutanoate | 68983-11-9 | 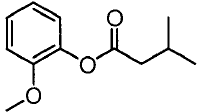 |
| Guaiacol propionate | 2-methoxyphenyl propionate | 7598-60-9 | 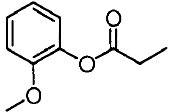 |
| Guaiacol 2-ethyl butyrate | 2-methoxyphenyl 2-ethylbutanoate | 723758-83-6 | 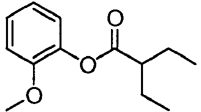 |
| Guaiacol butyrate | 2-methoxyphenyl butyrate | 4112-92-9 | 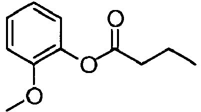 |
| Guaiacol 3-methyl pentanoate | 2-methoxyphenyl 3-methylpentanoate | No CAS | 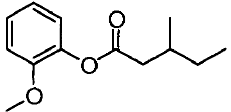 |
| Creosol propionate | 2-methoxy-4-methylphenyl propionate | 108439-89-0 | 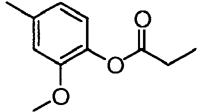 |
| Creosol butyrate | 2-methoxy-4-methylphenyl butyrate | No CAS | 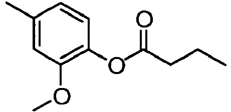 |
| Creosol valerate | 2-methoxy-4-methylphenyl valerate | No CAS | 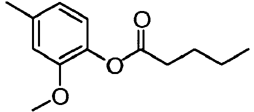 |
| Guaiacol succinate | 2-methoxyphenyl succinate | 39560-36-6 | 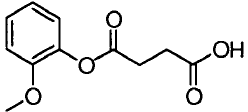 |
| Guaiacol lactate | 2-methoxyphenyl lactate | 59643-85-5 | 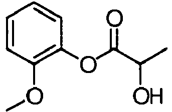 |
| Creosol acetate | 2-methoxy-4-methylphenyl acetate | 879-67-4 | 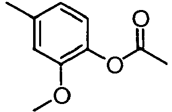 |
| Creosol 2-methyl butanoate | 2-methoxy-4-methylphenyl 2-methylbutanoate | No CAS | 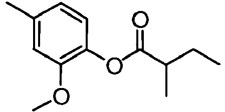 |
| Creosol 2-methyl pentanoate | 2-methoxy-4-methylphenyl 2-methylpentanoate | No CAS | 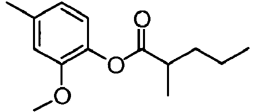 |
| Guaiacol tetrahydro furan-2-carboxylate | 2-methoxyphenyl tetrahydrofuran-2-carboxylate | No CAS | 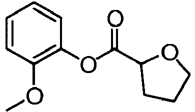 |
| Guaiacol valerate | 2-methoxyphenyl valerate | 531-39-5 | 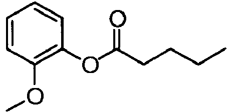 |
| Guaiacol hexanoate | 2-methoxyphenyl hexanoate | 613-00-3 | 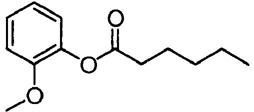 |

**Table 2**

| **Common Name** | **Chemical Name** | **CAS** | **Structure** |
|---|---|---|---|
| Guaiacol isobutytrate | 2-methoxyphenyl isobutyrate | 723759-62-4 | 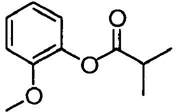 |
| Guaiacol 2-methyl butyrate | 2-methoxyphenyl 2-methylbutyrate | 379692-90-7 | 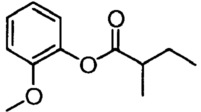 |
| Guaiacol tiglate | 2-methoxyphenyl tiglate | No CAS | 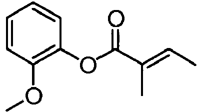 |
| Creosol hexanoate | 2-methoxy-4-methylphenyl hexanoate | No CAS | 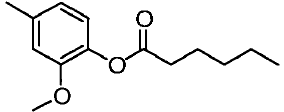 |
| Cresosol ethyl-2-butyrate | 2-methoxy-4-methylphenyl ethyl-2-butyrate | No CAS | 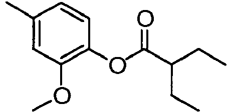 |
| Creosol 4-methyl pentanoate | 2-methoxy-4-methylphenyl 4-methylpentanoate | No CAS | 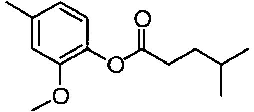 |

**Table 3**

| **Common Name** | **Chemical Name** | **CAS** | **Structure** |
|---|---|---|---|
| Creosol isobutyrate | 2-methoxy-4-methylphenyl isobutyrate | No CAS | 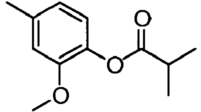 |
| Creosol isovalerate | 2-methoxy-4-methylphenyl isovalerate | No CAS | 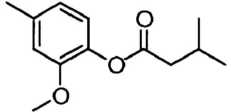 |
| Creosol 3-methyl pentanoate | 2-methoxy-4-methylphenyl 3-methyl pentanoate | No CAS | 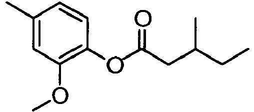 |
| Guaiacol 2-methyl pentanoate | 2-methoxyphenyl 2-methylpentanoate | No CAS | 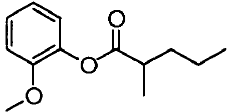 |
| Guaiacol isocaproate | 2-methoxyphenyl 4-methylpentanoate | 725736-75-4 | 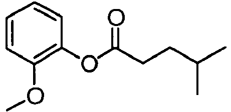 |
| Guaiacol heptanoate | 2-methoxyphenyl heptanoate | No CAS | 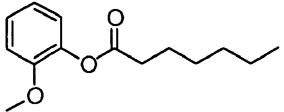 |

The compounds may be used in any orally-receivable product. There is therefore additionally provided a vanilla beany-flavored orally-receivable product comprising a product base in which is included at least one compound selected from the group consisting of those according to Formula I, as hereinabove described.

Examples of such products include, but are not limited to, the following:
Beverages, including soft drinks and alcoholic beverages, tea, coffee and juices.
Sweet goods, including baked products such as cakes, cookies and biscuits, pies, cereals, cereal bars; confectionery, such as hard candy, chewing gum, chocolates, soft candies and jellies.
Dairy products, including milk, ice cream and yogurt.
Savory products, including snack foods, dressings, dips, potato chip snacks.
Medicinal products, such as syrups, chewable tablets.
Dentifrices, including toothpastes, tooth powders, tooth gels, mouthwashes and edible films.

The method will now be further described with reference to the following examples. These describe particular embodiments and are not intended to be in any way limiting.

### Example 1

### Preparation of guaiacol lactate

To a solution of lactic acid methyl ester 1 (25g, 9.6 mmol) and DMF (50ml, 2ml/g solvent) was added *tert*-butyldimethylsilyl chloride (43.4g, 288.2 mmol), followed by imidazole (40.8g, 144.2 mmol). The reaction was stirred at room temperature overnight and then quenched with water. The mixture was extracted with ethyl acetate (3x50 ml). The combined organic layers were washed with 1 N HCl, water and saturated NaCl, dried (Na₂SO₄), filtered, and concentrated. The crude was purified by flash column chromatography eluting with 100% hexane to obtain 41.8g (80%) of product ("Product 2").

To a mixture of Product 2 (3g, 13.8 mmol) and THF (130ml) at 0°C was added 1.13g (27.5 mmol) of LiOH in 130 ml water. After being stirred at RT for about 4 h, the THF was removed under vacuum. The resulting aqueous mixture was washed with EtOAc (2×50ml). The EtOAc layers were extracted with saturated NaHCO₃. The aqueous layers were combined, acidified to pH=5 with saturated KHSO₄, and then extracted with EtOAc. The resulting organic layers were combined, dried and concentrated to afford 2.49g (89%) of product as clear oil ("Product 3"). Product 3 may be used without further purification.

To a solution of Product 3 (2.49g, 12.2 mmol) in 50 ml CH₂Cl₂ was treated DCC at 0°C. After the mixture was stirred for 10 min, guaiacol (2.3g, 18.5 mmol) and DMAP (0.15g, 1.2 mmol) were added to the reaction mixture at 0°C. The reaction was stirred at room temperature for over night. More methylene chloride was added to the reaction mixture. The reaction mixture was then washed with 1N HCl and water, dried over Na₂SO₄ and concentrated in *vacuo.* The crude product was purified on silica with 5% EtOAc/Hex and gave 1.74g (46%) of product ("Product 4").

Product 4 (1.74g, 5.6 mmol) was dissolved in 20 ml CH₃CN, cooled to 0°C, HF/Pyr (2 ml) was added, then the ice bath was removed and the reaction was stirred at room temperature for 2.5 hrs. Water was added into the reaction mixture and extracted back with EtOAc (3×50ml), washed the organic layers with water, brine, dried and concentrated. Flash chromatography (20%EtOAc/Hex) yielded 0.93g (86%) of final product as clear oil.

### Example 2

### Preparation of guaiacol succinate

Guaiacol (2.23g, 17.9 mmol), succinic anhydride (2.7g, 26.9 mmol), and 4-(dimethylamino)pyridine (50mg, 0.40 mmol) were dissolved in 10 ml pyridine and left for 2 days at room temperature. After addition of 50 ml of CH₂Cl₂, the resulting solution was washed with 1 N HCl (3x50 ml), water, brine, dried (Na₂SO₄), and concentrated. Flash chromatography (20% EtOAc/Hex to 100 % EtOAc) on silica gel yielded 1.47g (37%) of white solid as final product.

### Example 3

### Preparation of 2-Methoxyphenyl tetrahydrofuran-2-carboxylate

2-Tetrahydrofuroic acid (16g, 137.8 mmol) was dissolved in 70 ml CH₂Cl₂ and was treated with 200 ml of 2 M solution of oxalyl chloride in CH₂Cl₂ and 0.2 ml of DMF at room temperature. The reaction was stirred at room temperature for 2.5 h. An excess amount of oxalyl chloride was removed under vacuo. To a solution of guaiacol (5g, 40.3 mmol) in 50 mL CH₂Cl₂ was added freshly prepared tetrahydrofuran-2-carbonyl chloride (8.1g, 60.4 mmol) and 6.6 ml (100.6 mmol) of pyridine at room temperature. The resulting mixture was stirred at room temperature for 15 min, quenched with MeOH. The reaction mixture was poured into 50 mL 1 N HCl. The aqueous layer was back-extracted with CH₂Cl₂ (3x50mL), and the organic layers were combined and washed with 1 N HCl, water, saturated NaCl, dried (Na₂SO₄) and concentrated. Purification via flash chromatography on SiO₂ (20% EtOAc/Hex) gave 8.95g (63%) of product.

### Example 4

### Alcoholic beverage with vanilla flavor

The compounds listed in Table 4 were added to the following alcoholic beverage base at a concentration of 0.5 or 1 ppm.

| **Ingredient** | **Amount** |
|---|---|
| Water | 852.78 mL |
| Sodium Benzoate | 0.25 g |
| Potassium Sorbate | 0.15 g |
| Sodium Citrate | 0.3 g |
| Ethanol 190 proof | 52.6 mL |
| Vanilla flavor V1* (5%) | 0.1 g |
| High Fructose Corn Syrup-55 | 95 mL |

| | |
|---|---|
| *commercially-available vanilla flavor ex Givaudan Flavors Corp. | |

The flavor profile was evaluated by three vanilla flavorists. Comparing the resulting composition with the aroma and taste profile of the starting beverage, the vanilla flavor was found to be enhanced and/or improved. The enhancing effects are listed in Table 4.

**Table 4**

| **Compound** | **Flavor Profile Description in the Beverage Base** |
|---|---|
| Creosol Acetate | finish is a little more brown and fatty, slightly smoky, slightly more vanillin, improvement is a little subtle |
| Guaiacol tetrahydrofuran-2-carboxylate | more smoky and dark, interesting. |
| Guaiacol valerate | beany, fruity finish, added guaiacol-like aroma, but sweeter |
| Guaiacol octanoate | helps aroma, taste is a little more brown, resinous and slightly phenolic |

### Example 5

### Cola Beverage with vanilla flavor

The compounds listed in Table 5 were added to the following cola beverage base at a concentration of 0.5 or 1 ppm.

| **Ingredient** | **Amount** |
|---|---|
| Cola Syrup Base | 167 g |
| Vanilla Flavor V2* (5%) | 0.01 g |
| Carbonated Water (Schweppes) | 833 g |

| | |
|---|---|
| *commercially-available vanilla flavor ex Givaudan Flavors Corp. | |

The flavor profile was evaluated by three vanilla flavorists. Comparing the resulting composition with the aroma and taste profile of the starting beverage, the vanilla flavor was enhanced and/or improved. The enhancing effects are listed in Table 5:

**Table 5**

| **Compound** | **Amount (ppm)** | **Flavor Profile Description in the Cola Base** |
|---|---|---|
| Creosol Acetate | 0.5 | slightly sweeter ending, beany, phenolic, slightly sweeter |
| Guaiacol tetrahydrofuran-2-carboxylate | 0.5 | very slightly smoky, slightly brown beany, phenolic, slightly medicinal |
| | 1 | |
| Guaiacol valerate | 0.5 | beany, phenolic, medicinal, a little more brown note, some sweet character at the end, nondescript nice background note |
| | 1 | |
| Guaiacol octanoate | 0.5 | Slightly sweetening, very slightly fatty/resinous |

### Example 8 6

### Cookie with vanilla flavor

Compounds listed in Table 6 were added to the following cookie base at a concentration of 200 ppm.

| **Ingredient** | **Amount** |
|---|---|
| Cookie Base | 998 g |
| Vanilla Flavor V1 (5%) | 2 g |

The flavor profile was evaluated by three vanilla flavorists. Comparing the resulting composition with the aroma and taste profile of the cookie base, the vanilla flavor was enhanced and/or improved. The enhancing effects are listed in Table 6.

**Table 6**

| **Compound** | **Flavor Profile Description in the Cookie Base** |
|---|---|
| Creosol Acetate | Beany, resin, fatty, creamy, vanilla-like, sweeter |
| Guaiacol tetrahydrofuran-2-carboxylate | Beany, smoky, resinous, phenolic/guaiacol-like |
| Guaiacol valerate | Better beany character, fruity |
| Guaiacol octanoate | Mild vanilla, smoothes flavor |

### Example 9 7

### Chewing gum with vanilla flavor

The compounds listed in Table 7 were added to the following chewing gum base at a concentration of 250 ppm.

| **Ingredient** | **Amount** |
|---|---|
| Gum Base | 995 g |
| Vanilla Flavor V1 (5%) | 5 g |

The flavor profile was evaluated by three vanilla flavorists. Comparing the resulting composition with the aroma and taste profile of the chewing gum base, the vanilla flavor was enhanced and/or improved. The enhancing effects are listed in Table 7.

**Table 7**

| **Compound** | **Flavor Profile Description in the Gum Base** |
|---|---|
| Creosol Acetate | Slightly brown, more sweet, caramel or cocoa like, good flavor |
| Guaiacol tetrahydrofuran-2-carboxylate | Nice beany, brown, slightly fatty, resinous |

### Example 8

### Milk with vanilla flavor

The compounds listed in Table 8 were added to the following milk base at a concentration of 20 ppm.

| **Ingredient** | **Amount** |
|---|---|
| Whole Milk | 908.82 g |
| Sucrose | 90.88 g |
| Vanilla Beany Flavor V1 (5%) | 0.30 g |

The flavor profile was evaluated by three vanilla flavorists. Comparing the resulting composition with the aroma and taste profile of the milk base, the vanilla flavor was enhanced and/or improved. The enhancing effects are listed in Table 8.

**Table 8**

| **Compound** | **Flavor Profile Description in the Milk Base** |
|---|---|
| Creosol Acetate | sweeter, moot on guaiacol, rummy, smoky, beany |
| Guaiacol valerate | softens smoky notes, slightly caramel-like, beany, dried fruit/raisin, nice |

### Example 9

### Yogurt with vanilla flavour

The compounds listed in Table 9 were added to the following yogurt base at a concentration of 20 ppm.

| **Ingredient** | **Amount** |
|---|---|
| Plain Yogurt | 908.82 g |
| Sucrose | 90.88 g |
| Vanilla Flavor V2 (5%) | 0.3 g |

The flavor profile was evaluated by three vanilla flavorists. Comparing the resulting composition with the aroma and taste profile of the yogurt base, the vanilla flavor was enhanced and/or improved. The enhancing effects are listed in Table 9.

**Table 9**

| **Compound** | **Flavor Profile Description in the Yogurt Base** |
|---|---|
| Creosol Acetate | Sweeter, beany, nice, covers some of the phenolic notes of reference |
| Guaiacol tetrahydrofuran-2-carboxylate | Beany, smoky, brown, slightly caramelic, raisin, sweet |
| Guaiacol valerate | Very smoky, rummy, beany, slightly resinous, slightly more brown |
| Guaiacol octanoate | Finish has nice beany note, sweeter, dried fruit |

### Example 10

### Mouthwash with vanilla flavor

The compounds listed in Table ***10*** were added to the following mouthwash base at a concentration of 50 ppm.

| **Ingredient** | **Amount** |
|---|---|
| Mint flavored mouthwash | 19.98 g |
| Water | 979.02 g |
| Vanilla Flavor V1 (5%) | 1 g |

The flavor profile was evaluated by three vanilla flavorists. Comparing the resulting composition with the aroma and taste profile of the mouthwash base, the vanilla flavor was enhanced and/or improved. The enhancing effects are listed in Table ***10***.

**Table 10**

| **Compound** | **Flavor Profile Description in the Mouthwash Base** |
|---|---|
| Creosol Acetate | A little more brown, sweeter, improved beany |
| Guaiacol octanoate | Beany, slightly sweet, doughy, balanced |

## Claims

1. A method of providing vanilla beany flavor in an orally-receivable product, comprising adding to a product base at least one compound selected from those according to Formula I 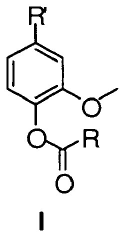 in which R' is selected from the group consisting of hydrogen and methyl, and R is selected from the group consisting of hydrogen, methyl, ethyl, propyl, isopropyl, n-butyl, 1- and 2-methylpropyl, n-pentyl, 1-, 2- and 3-methylbutyl, 1-, 2-, 3-, and 4-methylpentyl, 1-methylbut-2-ene, hydroxyethyl, ethylcarboxy, 2-tetrahydrofuranyl and a group of the formula VII

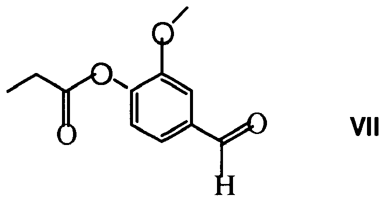

2. A method according to claim 1, in which the compound is selected from the group consisting of guaiacol propionate, guaiacol butyrate, guaiacol isobutyrate, guaiacol 3-methyl pentanoate, guaiacol tiglate ,guaiacol 2-methyl pentanoate and guaiacol tetrahydro furan-2-carboxylate

3. A compound of the formula III 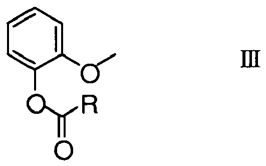 in which R is a moiety selected from the group consisting of 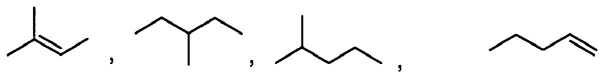 and

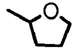

4. A compound according to claim 3, selected from the group consisting of guaiacol 3-methyl pentanoate, guaiacol tiglate, guaiacol 2-methyl pentanoate, and guaiacol tetrahydro furan-2-carboxylate.

5. A compound of the formula IV 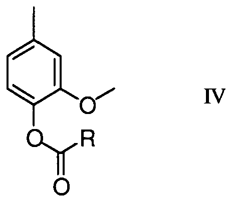 in which R is a moiety selected from the group consisting of 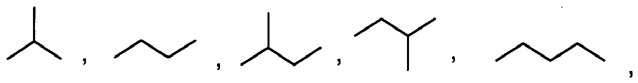 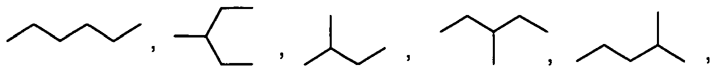 and

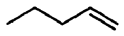

6. A compound according to claim 5, selected from the group consisting of creosol butyrate, creosol valerate, creosol 2-methyl butanoate, creosol 2-methyl pentanoate, creosol hexanoate, cresosol ethyl-2-butyrate, creosol 4-methyl pentanoate, creosol isobutyrate, creosol isovalerate and creosol 3-methyl pentanoate.

7. A vanilla beany-flavored orally-receivable product comprising a product base in which is included at least one compound selected from the group of Formula I 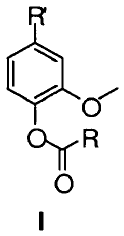 in which R' is selected from the group consisting of hydrogen and methyl, and R is selected from the group consisting of hydrogen, methyl, ethyl, propyl, isopropyl, n-butyl, 1- and 2-methylpropyl, n-pentyl, 1-, 2- and 3-methylbutyl, 1-, 2-, 3-, and 4-methylpentyl, 1-methylbut-2-ene, hydroxyethyl, ethylcarboxy, 2-tetrahydrofuranyl and a group of the formula VII

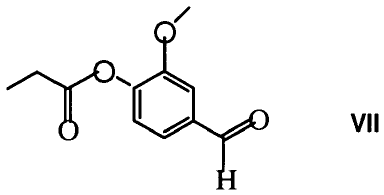

## Patentansprüche

1. Verfahren zum Bereitstellen von Vanilleschotenaroma in einem oral annehmbaren Produkt, umfassend das Zugeben von wenigstens einer Verbindung ausgewählt aus jenen gemäß Formel I zu einer Produktgrundlage, 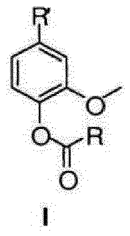 wobei R' ausgewählt ist aus der Gruppe bestehend aus Wasserstoff und Methyl und R ausgewählt ist aus der Gruppe bestehend aus Wasserstoff, Methyl, Ethyl, Propyl, Isopropyl n-Butyl, 1- und 2-Methylpropyl, n-Pentyl, 1-, 2- und 3-Methylbutyl, 1-, 2-, 3- und 4-Methylpentyl, 1-Methylbut-2-en, Hydroxyethyl, Ethylcarboxy, 2-Tetrahydrofuranyl und einer Gruppe der Formel VII

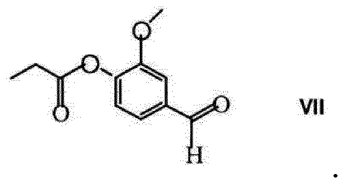

2. Verfahren gemäß Anspruch 1, wobei die Verbindung ausgewählt ist aus der Gruppe bestehend aus Guajacolpropionat, Guajacolbutyrat, Guajacolisobutyrat, Guajacol-3-methylpentanoat, Guajacoltiglat, Guajacol-2-methylpentanoat und Guajacoltetrahydrofuran-2-carboxylat.

3. Verbindung der Formel III, 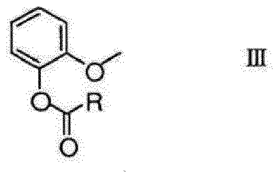 wobei R eine Einheit ausgewählt aus der Gruppe bestehend aus 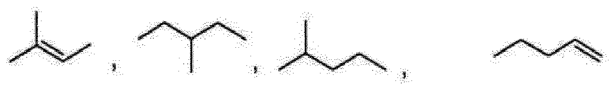 und 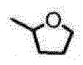 ist.

4. Verbindung gemäß Anspruch 3, ausgewählt aus der Gruppe bestehend aus Guajacol-3-methylpentanoat, Guajacoltiglat, Guajacol-2-methylpentanoat und Guajacoltetrahydrofuran-2-carboxylat.

5. Verbindung der Formel IV, 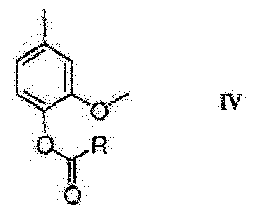 wobei R eine Einheit ausgewählt aus der Gruppe bestehend aus 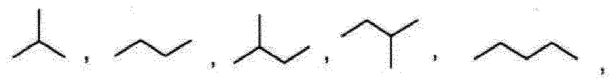 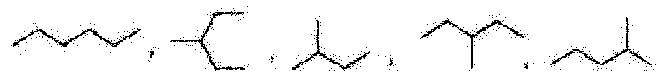 und 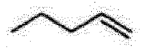 ist.

6. Verbindung gemäß Anspruch 5, ausgewählt aus der Gruppe bestehend aus Creosolbutyrat, Creosolvalerat, Creosol-2-methylbutanoat, Creosol-2-methylpentanoat, Creosolhexanoat, Creosolethyl-2-butyrat, Creosol-4-methylpentanoat, Creosolisobutyrat, Creosolisovalerat und Creosol-3-methylpentanoat.

7. Vanilleschoten-aromatisiertes, oral annehmbares Produkt, umfassend eine Produktgrundlage, in der wenigstens eine Verbindung ausgewählt aus der Gruppe von Formel I eingeschlossen ist, 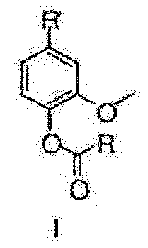 wobei R' ausgewählt ist aus der Gruppe bestehend aus Wasserstoff und Methyl, und R ausgewählt ist aus der Gruppe bestehend aus Wasserstoff, Methyl, Ethyl, Propyl, Isopropyl, n-Butyl, 1- und 2-Methylpropyl, n-Pentyl, 1-, 2- und 3-Methylbutyl, 1-, 2-, 3- und 4-Methylpentyl, 1-Methylbut-2-en, Hydroxyethyl, Ethylcarboxy, 2-Tetrahydrofuranyl und einer Gruppe der Formel VII

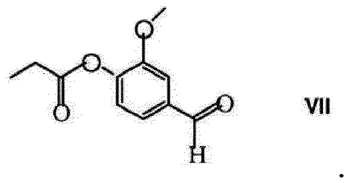

## Revendications

1. Méthode de fourniture d'un arôme de type gousse de vanille dans un produit recevable par voie orale, comprenant l'addition à une base de produit d'au moins un composé choisi parmi ceux répondant à la Formule I 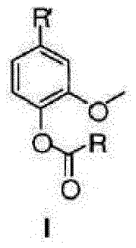 dans laquelle R' est choisi dans le groupe constitué par hydrogène et méthyle, et R est choisi dans le groupe constitué par hydrogène, méthyle, éthyle, propyle, isopropyle, n-butyle, 1- et 2-méthylpropyle, n-pentyle, 1-, 2- et 3-méthylbutyle, 1-, 2-, 3- et 4-méthylpentyle, 1-méthylbut-2-ène, hydroxyéthyle, éthylcarboxy, 2-tétrahydrofuranyle et un groupe de formule VII

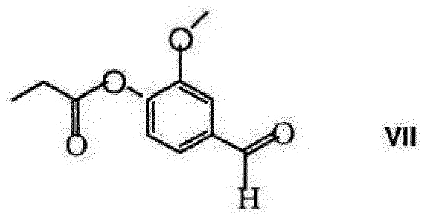

2. Méthode selon la revendication 1, dans laquelle le composé est choisi dans le groupe constitué par le propionate de gaïacol, le butyrate de gaïacol, l'isobutyrate de gaïacol, le 3-méthylpentanoate de gaïacol, le tiglate de gaïacol, le 2-méthylpentanoate de gaïacol et le tétrahydrofuran-2-carboxylate de gaïacol.

3. Composé de formule III 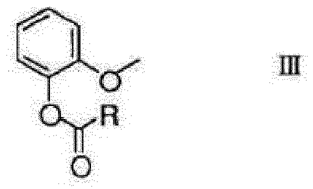 dans laquelle R est un motif choisi dans le groupe constitué par 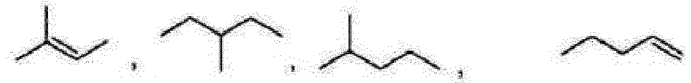 et

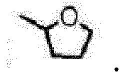

4. Composé selon la revendication 3, choisi dans le groupe constitué par le 3-méthylpentanoate de gaïacol, le tiglate de gaïacol, le 2-méthylpentanoate de gaïacol et le tétrahydrofuran-2-carboxylate de gaïacol.

5. Composé de formule IV 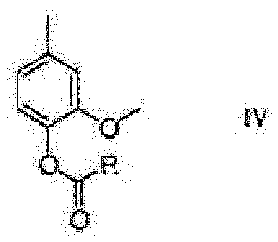 dans laquelle R est un motif choisi dans le groupe constitué par 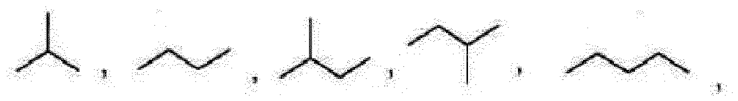 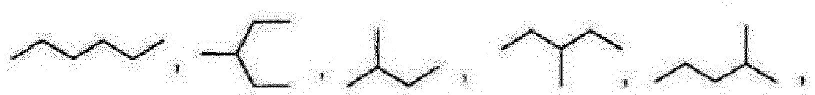 et

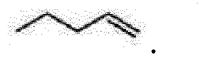

6. Composé selon la revendication 5, choisi dans le groupe constitué par le butyrate de créosol, le valérate de créosol, le 2-méthylbutanoate de créosol, le 2-méthylpentanoate de créosol, l'hexanoate de créosol, l'éthyl-2-butanoate de créosol, le 4-méthylpentanoate de créosol, l'isobutyrate de créosol et le 3-méthylpentanoate de créosol.

7. Produit recevable par voie orale à arôme de type gousse de vanille, comprenant une base de produit dans laquelle est incorporé au moins un composé choisi dans le groupe de Formule I 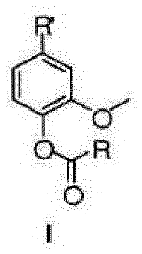 dans laquelle R' est choisi dans le groupe constitué par hydrogène et méthyle, et R est choisi dans le groupe constitué par hydrogène, méthyle, éthyle, propyle, isopropyle, n-butyle, 1- et 2-méthylpropyle, n-pentyle, 1-, 2- et 3-méthylbutyle, 1-, 2-, 3- et 4-méthylpentyle, 1-méthylbut-2-ène, hydroxyéthyle, éthylcarboxy, 2-tétrahydrofuranyle et un groupe de formule VII
